Europäisches Patentamt

European Patent Office

Office européen des brevets

(11) Publication number: **0 137 633 B1**

# EUROPEAN PATENT SPECIFICATION

(45) Date of publication of patent specification: **11.12.91** (51) Int. Cl.5: **C12N 15/15**, C12N 15/72

(21) Application number: **84305466.9**

(22) Date of filing: **10.08.84**

(54) **Method of expressing alpha-1-antitrypsin in bacteria and its use in therapeutic formulations, and vectors and bacteria for such method and their production.**

(30) Priority: **10.08.83 US 522190**

(43) Date of publication of application:
**17.04.85 Bulletin 85/16**

(45) Publication of the grant of the patent:
**11.12.91 Bulletin 91/50**

(84) Designated Contracting States:
**AT BE CH DE FR GB IT LI LU NL SE**

(56) References cited:
**EP-A- 0 114 777**

**PROCEEDINGS OF THE NATIONAL ACADEMY
OF SCIENCES OF THE UNITED STATES OF
AMERICA, vol. 78, no. 11, November 1981, Baltimore, US; K.KURACHI et al, "Cloning and
sequence of c DNA coding for
alpha1-antitrypsin", pp. 6826-6830**

**BIOCHEMICAL AND BIOPHYSICAL RE-
SEARCH COMMUNICATIONS, vol. 103, 1981,
New York/London/Toronto/Sydney/San
francisco/Tokyo; T. CHANDRA et al,
"Induction of alpha-antitrypsin m RNA and
cloning of its c DNA", pp. 751-758**

**METHODS IN ENZYMOLOGY, vol. 101, Re-
combinant DNA, part C, 1983, Academic
Press, New York etc.; J. MESSING, "New M13
Vectors for Cloning", pp. 20-78**

(73) Proprietor: **ZYMOGENETICS, INC.
2121 North 35th Street
Seattle Washington 98103(US)**

(72) Inventor: **Parker, Michael Lee
4613 229th Avenue
Redmond Washington 98025(US)**
Inventor: **Kawasaki, Glenn Hitoshi
1547 16th Avenue East
Seattle Washington 98112(US)**

(74) Representative: **Allard, Susan Joyce et al
BOULT, WADE & TENNANT, 27 Furnival
Street
London EC4A 1PQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to a method of expressing mammalian alpha-1-antitrypsin in bacteria and to the protein product expressed thereby.

Alpha-1-antitrypsin is a protease inhibitor present in mammalian blood whose apparently major physiological function is to inhibit elastase, a potent protease which hydrolyzes structural proteins. The alpha-1-antitrypsin also inhibits other serine proteases. The normal plasma level of alpha-1-antitrypsin is about 2 mg./ml. A low level of alpha-1-antitrypsin in the blood may be associated with chronic obstructive pulmonary emphysema and infantile liver cirrhosis. Under many inflammatory conditions an acute-phase response is initiated and the concentration of alpha-1-antitrypsin is substantially increased. In order to study and treat alpha-1-antitrypsin deficiency and to examine the mechanism of acute phase response, it is therefore desirable to have a pure alpha-1-antitrypsin protein. In particular, it is desirable to have a source of antitrypsin protein produced by bacteria through genetic engineering techniques.

It is therefore an object of the present invention to provide a method for producing alpha-1-antitrypsin in bacteria.

It is a further object of the present invention to provide a pure alpha-1-antitrypsin polypeptide which has been produced in bacteria by genetic engineering techniques.

In the accompanying figures:

FIG. 1 is a diagram of the steps for converting the vector M13 mp10 to a linear fragment adapted to receive the trp-lac promotor.

FIG. 2 is a diagram of the steps for isolating the trp-lac promotor fragment from plasmid pDR540.

FIG. 3 is a diagram of the steps for fusing the final fragments from FIGs. 1 and 2 and converting same to vector m13TAC.

FIG. 4 is a diagram of gene coding for the predominant form of human alpha-1-antitrypsin.

FIG. 5A is the polylinker DNA sequence and corresponding amino acid sequence at the start of the lacZ gene in pUC13.

FIG. 5B is the restriction map of plasmid pUC-alpha-1.

FIG. 6 is a restriction map of plasmid M13$\alpha_1$AT.

FIG. 7 shows the results of the elastase binding assay of lacZ-$\alpha_1$AT fusion protein prepared according to Example 3.

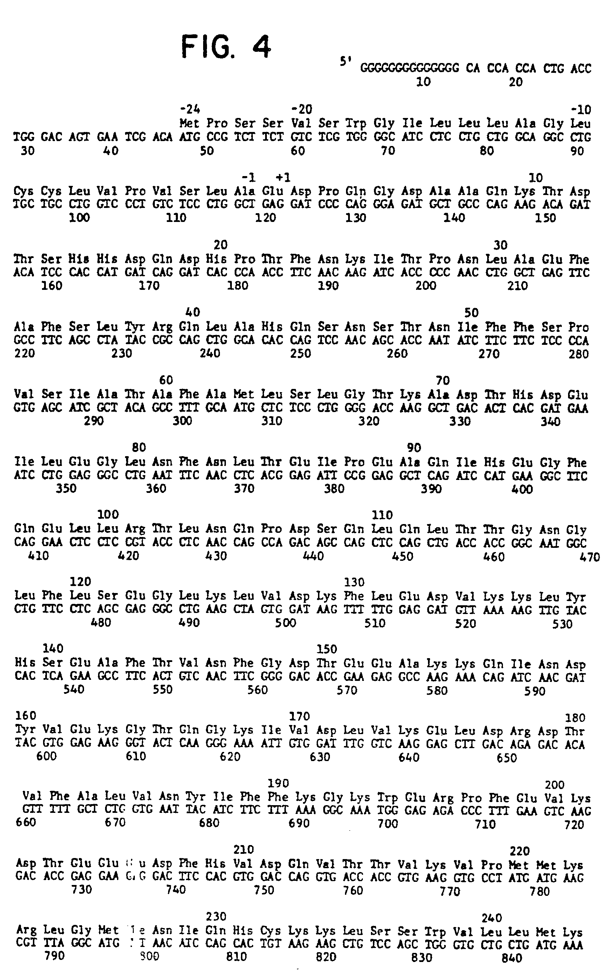

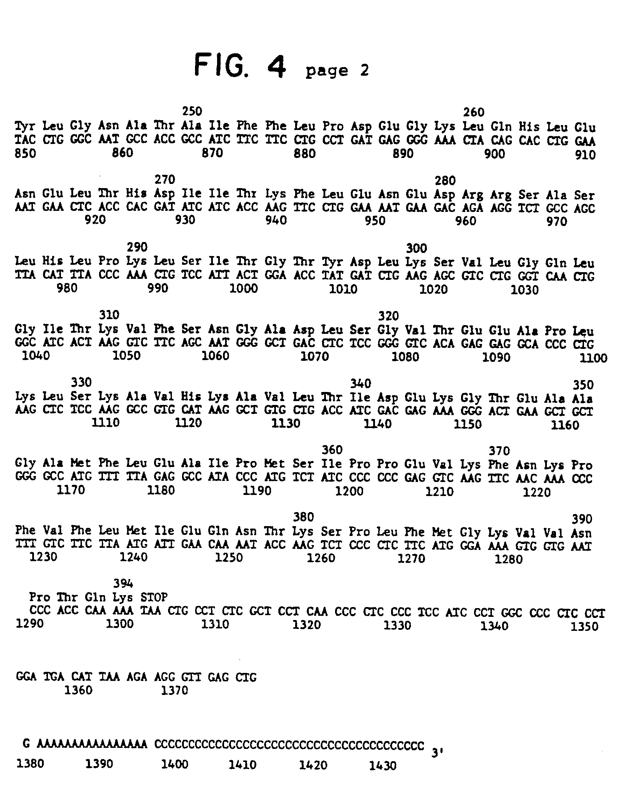

The sequencing of chromosomal DNA (cDNA) coding for alpha-1-antitrypsin has been described by Kurachi et al., Proc.Natl.Acad.Sci.U.S.A., 78, 6826-6830 (1981) and by Chandra et al., Biochem.Biophys.Res.Comm., 103, 751-758 (1981). A primate gene for alpha-1-antitrypsin may be obtained by DNA cloning methods described by Chandra et al., ibid. Also, the gene coding for the predominant form of human alpha-1-antitrypsin, isolated from a human cDNA library is shown in FIG. 4.

The present invention is directed to a method for producing alpha-1-antitrypsin (hereinafter AT) in bacteria by inserting a cDNA coding sequence for AT into an expression vector, which, in bacteria, will express a polypeptide having the protease inhibition activity of mammalian AT. The DNA transfer vectors contain, in addition to the cDNA coding segment for mammalian AT, appropriately positioned translational start and stop signals and a Trp-lac or lac promoter segment and genes for phenotypic selection. The cDNA segments containing coding sequences for alpha-1-antitrypsin are known as described above. Preferably the gene coding for the predominant form of human alpha-1-antitrypsin may be utilized, the sequence of which is set forth in accompanying FIG. 4. However, the expression of less antigenic and/or more stable mutant forms of AT may also be useful particularly for subjects having variant forms of AT. The predominant human AT coding sequence may be isolated from a cDNA library by using the baboon AT gene (Kurachi, et al., ibid.) as a hybridization probe.

Vectors according to the present invention are derived from the M13 cloning vectors, described by Messing, (Methods in Enzymology, 101 (1983)). A particularly preferred vector is phage M13 mp10, described by Messing et al., ibid, and commercially available from P-L Biochemicals. This vector contains EcoR1 and BamHI restriction sites in the linker sequence, and a unique AvaII site in the lacI region. To enhance the expression of AT by this vector in bacteria, M13 mp10 is modified by insertion of a hybrid trp-lac promoter obtainable from the plasmid pDR540. This latter plasmid is described by Russell et al., Gene, 20, 231-243 (1982) and is commercially available from P-L Biochemicals.

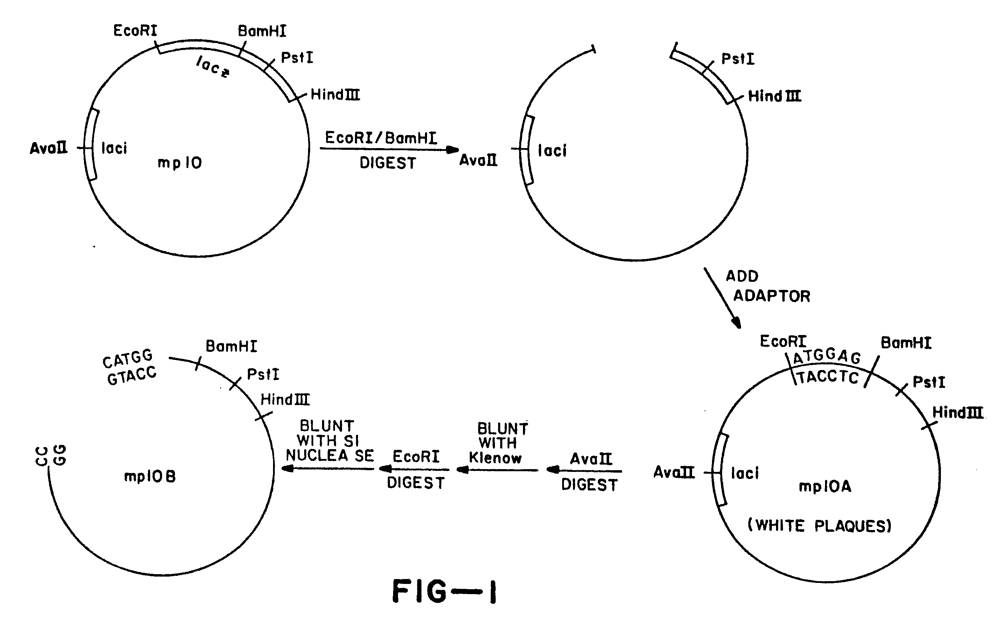

Referring to FIG. 1, the preferred DNA transfer vector according to the present invention is prepared by digesting the phage M13 mp10 with EcoRI and BamHI. A synthetic DNA adaptor, purchased from P-L Biochemicals, having the following structure and lacking the five prime phosphates

AATTCATGGAG
GTACCTCCTAG

is ligated cnto the resultant sticky ends to form the construct mp10A shown in FIG. 1. The construct mp10A thus contains EcoRI and BamHI restriction sites about a sequence including ATGGAG which provides the initiation codon and the first amino acid (Glu) codon for the AT gene. The substitution of the adaptor for the region between the original EcoRI and BamHI sites of mp10 destroys the lac operon reading frame and the resulting transfectants give white plaques.

The vector mp10A is digested with AvaII and the sticky ends filled using the Klenow fragment of DNA polymerase. This is followed by digestion with EcoRI, and removal of the sticky end using S1 nuclease. The resultant blunt ended fragment is shown in FIG. 1 as mp10B.

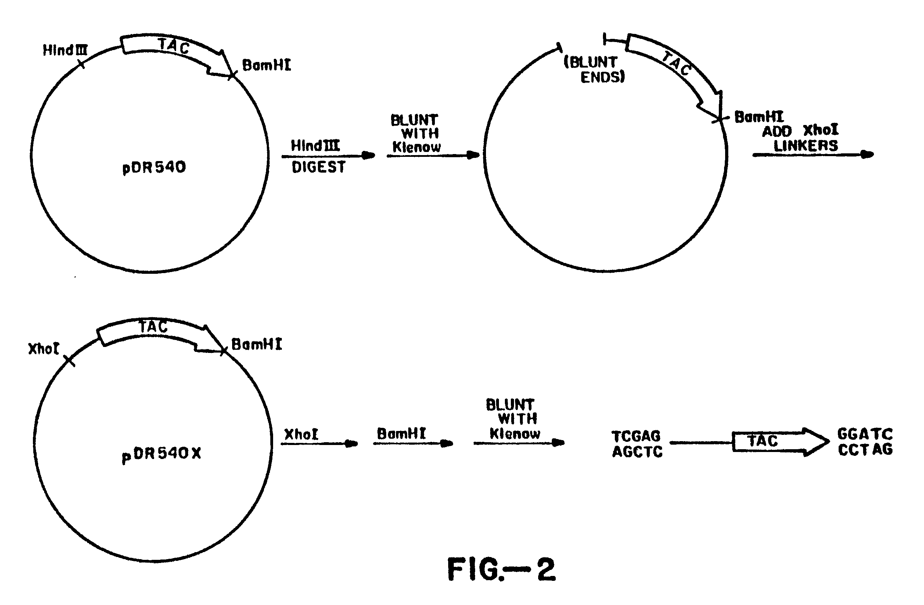

Referring to FIG. 2, a DNA fragment comprising the trp-lac promoter is removed from pDR540. The plasmid pDR540 is cut with HindIII and the sticky ends are filled with Klenow polymerase. Linkers having the sequence CCTCGAGG are ligated to the blunt ends and excess linkers are removed by digestion with XhoI. The resulting construct, known as pDR540X, contains an XhoI site in place of the HindIII site of pDR540. Digesting pDR540X with XhoI and BamHI, followed by blunting the ends using Klenow fragment, yields a fragment containing the trp-lac promoter (TAC) and Shine-Dalgarno sequence.

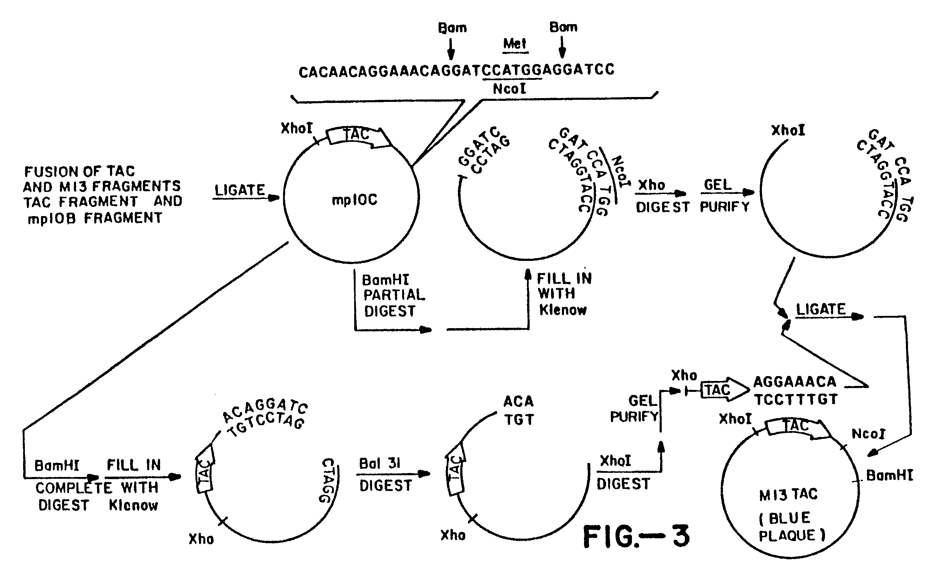

Referring to FIG. 3, the above described fragment containing the trp-lac promoter is inserted into the mp10B fragment (the final construct shown in FIG. 1), producing the hybrid phage mp10C. Ligation of the blunted AvaII end of mp10B to the blunted XhoI end of the TAC containing fragment regenerates an XhoI site at the junction. Ligation of the blunted BamHI site of the TAC fragment to the blunted EcoRI end of mp10B creates an NcoI site (CCATGG) at this junction. The proper orientation of the fragment may be screened for by the formation of blue plaques. The phage mp10C contains a second BamHI site located upstream of the ATG initiation codon which must be removed to facilitate insertion of the AT gene into the original BamHI site. To remove this extraneous BamHI site, mp10C is subjected to two digestions with BamHI. The first, a partial digestion, is followed by filling in the sticky ends with Klenow polymerase, digesting with XhoI, and purifying on an agarose gel. The proper fragment is identified as the one containing the NcoI restriction site. The second BamHI digestion of mp10C is run to completion, the sticky ends are filled using Klenow polymerase and $\alpha$-$^{32}$P-dNTP's are used to facilitate monitoring of subsequent manicuring of the blunt ends by Bal 31 exonuclease. Five base pairs are removed from the labeled terminus by Bal 31 exonuclease, thereby eliminating the BamHI site. The sequence containing the promoter is removed with XhoI and gel purified. The mp10 and pDR540-derived fragments are ligated together, cloned into E.coli K12 (JM103) (Messing, J. et al. 1981. Nucleic Acids Res. 9:309, commercially available from P-L Biochemicals) and screened for NcoI sensitivity and formation of blue plaques. The resulting vector, shown in FIG. 3, is M13 TAC.

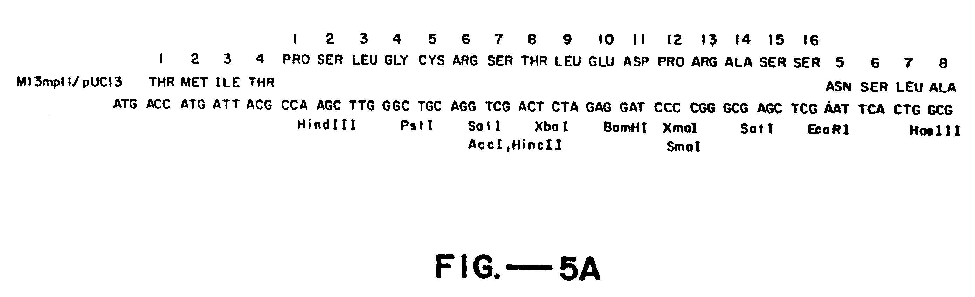

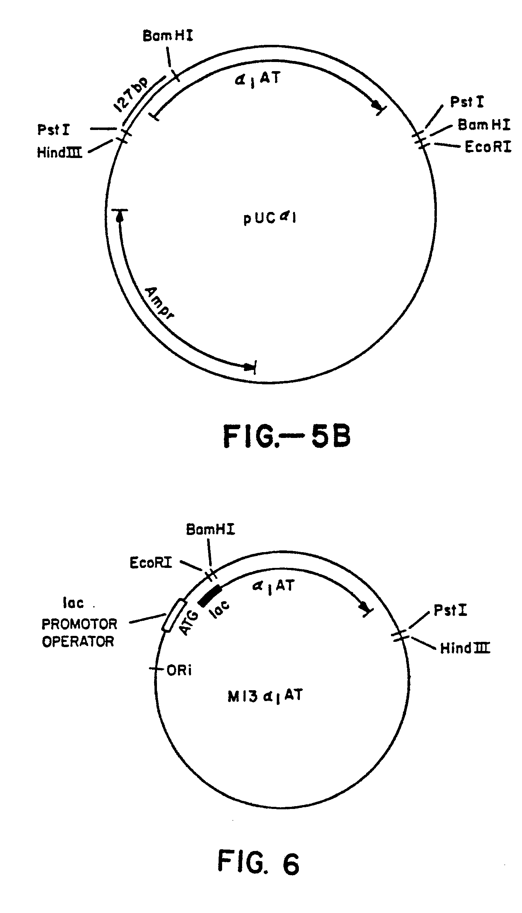

The AT sequence of FIG. 4 is flanked by PstI restriction sites as a result of being cloned into the PstI site of pBR322 (Kurachi, et al., ibid.). The AT gene may, therefore, be removed as a PstI fragment of approximately 1446 base pairs and may be inserted into the plasmid pUC13; (pUC13 is constructed as described for pUC8 and pUC9 in Messing, Meth. in Enzymology, 101, (1983), but containing the polylinker sequence shown in FIG. 5A at the start of the lacZ gene; see also Vieira and Messing, Gene 19, 259-268, (1982) to produce the plasmid pUC-$\alpha$1 (FIG. 5B). pUC-$\alpha$1 is digested with PstI and BamHI to produce a fragment containing AT coding sequences which may be inserted into a BamHI-PstI-digested M13 TAC vector. The protein expressed by the resulting recombinant phage starts with a Met followed by the sequence of mature AT. The recombinant phage may be used to transfect E.coli K12 (JM103). The cells are grown, infected, and induced according to the method of Slocombe et al., PNAS U.S.A., 79, 5455 (1982). Samples are collected at various times after induction and the cells harvested by a low speed centrifugation and resuspended in 1/20 volume of 50 mM tris pH 8.0, 30 mM NaCl, 10 mM EDTA. The supernatant is assayed for extracellular phage, and lysozyme is added to the bacterial suspension to a final concentration of 1 mg./ml. After 30 minutes at 4°C, the bacteria are lysed by several cycles of freeze-thaw and the cell debris is removed by centrifugation at 100,000 xg for 1 hour. The supernatant is treated with DNAse, and assayed for AT by ELISA and elastase binding assay.

EXAMPLE 1

ELISA Assay for AT

To assay for AT, 200 microliters of goat anti-alpha-1-antitrypsin (10 micrograms/ml in 0.1M Na₂CO₃ pH 9.8) that has been affinity purified is placed in each well of a 96 well microtiter culture plate at 37° C for two to four hours. This solution is removed and 200 microliters of phosphate buffered saline (PBS) pH 7.2 containing 1% BSA and .05% Tween 20 is added to each well at 37° C for two hours.

Test samples, including standards, are then added to each well and AT, if present, binds to the specific antibody. The wells are rinsed with PBS after 15-30 minutes of incubation. 200 microliters of rabbit anti-alpha-1-AT is added to each well in the presence of 1% BSA in PBS. After 15 minutes incubation, the solution is removed and the wells are rinsed with PBS.

A sample of 200 microliters of goat anti rabbit IgG coupled to alkaline phosphatase in 1% BSA and PBS are added to each well and incubated for 15 minutes. After rinsing the wells, 200 microliters of a substrate solution was added which contains 30 milligrams of dinitrophenyl phosphate dissolved in 50 ml of 0.1 m diethanolamine pH 9.6. A yellow color is allowed to develop (15-60 minutes) and intensity is recorded at 405 nm on a Titertek Multiscan detector. The yellow color is proportional to the amount of AT in the test solution.

## EXAMPLE 2

Elastase Binding Assay for AT

Solutions:

1. $0.1M\ Na_2CO_3$ pH9.7
2. 0.05% Tween 20, 0.05% sodium azide in phosphate buffered saline (PBS).
3. Solution 2 containing 1% bovine serum albumin (BSA).
4. 96ml diethanolamine, 56 mg $MgCl_2$, $H_2O$ to 1 liter, pH 9.8
5. Alkaline phosphatase substrate (p-nitrophenol phosphate, disodium) (Sigma Chemical Co.), 30 mg per 50 ml of solution 4.

To each well of 96 well microtiter plate is added $200\mu l$ of solution 1 containing $10\mu g$ elastase (Sigma Chemical Co.) per ml. The plate is incubated at 37° for 2 hours. The solution is then removed and the wells washed twice with $200\mu l$ of solution 2. $300\mu l$ of solution 3 is then added to each well and the plate is incubated for 2 hours at 37°, or overnight in a refrigerator. The solution is removed and the wells washed as above. Samples to be tested are diluted in solution 3, and $200\mu l$ of diluted sample are added per well. $200\mu l$ of solution 3 are added to all unused wells. The plate is incubated 30 minutes at 37°, then emptied and washed as above. To each well is added $200\mu l$ of rabbit antibody against AT, which has been diluted 1:1000 in solution 3. The plate is incubated 30 minutes at 37°, emptied and washed 3 times with solution 2. To each well is added $200\mu l$ of alkaline phosphatase coupled to goat antirabbit IgG, diluted 1:1000 in solution 3. The plate is incubated 30 minutes at 37° and washed 3 times with solution 2. $200\mu l$ of solution 5 is then added, the plate incubated at 37° until a yellow color appears (30-60 minutes), and the optical density of the solution at 405 nm is recorded.

## EXAMPLE 3

Expression of a lacZ-$\alpha_1$AT fusion protein

Plasmid pUC$\alpha$1 (FIG. 5B) is digested with BAM H1 and PST 1 and the 1320 bp fragment is purified from an agarose gel (Anal. Biochem. 103, 264 [1980]). This fragment is ligated into M13mp10 which had also been digested with BAM H1 and PST1. The resulting construction, M13$\alpha_1$AT, is shown in FIG. 6. Following the protocol of Slocombe et al., ibid., E. coli K12 (JM103) is grown, infected with M13$\alpha_1$AT and induced as described therein. Samples are collected at various times after induction and the cells are harvested by a low-speed centrifugation and resuspended in 1/20 volume of 50mM Tris pH 8.0, 30 mM NaCl and 10 mM EDTA. The supernatant is assayed for extracellular phage. Lysozyme is added to the bacterial suspension to a final concentration of 1 mg/ml. After 30 min. at 4 C the bacteria are lysed by several cycles of freeze-thawing and the cell debris is removed by centrifugation at 100,000 xg for one hour. The supernatant is assayed for $\alpha_1$AT by ELISA and elastase binding assay following the procedures of Examples 1 and 2, respectively. The ELISA assays in Table I show that the culture with the expression vector but without the gene for $\alpha_1$AT (JM1Ø3) does not produce any $\alpha_1$AT. Two cultures of JM1Ø3 containing the M13$\alpha_1$AT expression vector expressed Ø.Ø6% and Ø.18% AT.

Vector M13$\alpha_1$AT was deposited under accession number 40132 on 8th August 1984 with American Type Culture Collections (ATCC) of 12301 Parklawn Drive, Rockville, Maryland 20852, U.S.A.

TABLE I

| Culture | Microgram $\alpha_1$AT/ml | milligram protein/ml | %$\alpha_1$AT |
|---|---|---|---|
| 1) JM1Ø3 | Ø | 8.25 | Ø |
| 2) JM1Ø3 + M13$\alpha_1$AT | 1.1 | 1.75 | Ø.Ø6 |
| 3) JM1Ø3 + M13$\alpha_1$AT | 12.4 | 5.5 | Ø.18 |

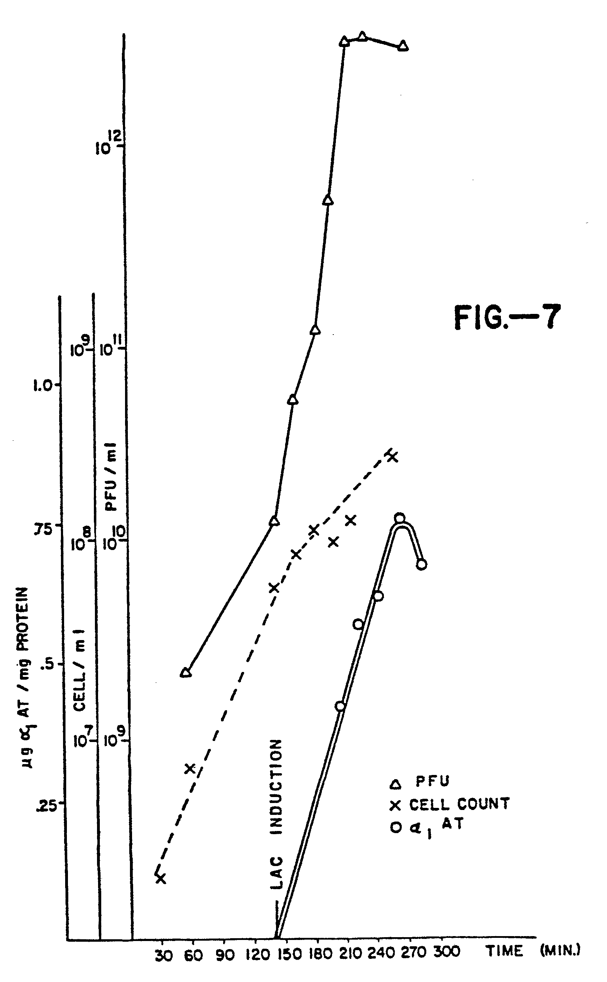

Figure 7 (pfu = plaque-forming units) illustrates the induction of AT in culture 3.

Similar results are obtained with the elastase binding assay indicating that $\alpha_1$AT expressed by the cultures is functionally active, and expression is under control of the inducible lac promotor.

Portions of the culture homogenates are passed through an immunadsorption column consisting of affinity purified goat anti-human $\alpha_1$AT coupled to Sepharose 4B. The column is washed extensively with phosphate buffered saline (PBS), pH 7.2, containing Ø.5 M NaCl. Any specifically immunoadsorbed material is then eluted from the column with 3M NaSCN. This material is dialyzed against PBS to remove the salt and then subjected to polyacrylamide gel electrophoresis in the presence of sodium dodecyl sulfate and mercaptoethanol. The results indicate that the $\alpha_1$AT expressed in E. coli is purified to homogeneity as a single polypeptide chain indicating that the expressed product is stable. In comparison to $\alpha_1$AT expressed in yeast (Ser. No. 489,406, filed April 28, 1983), the $\alpha_1$AT produced in E. coli is slightly higher in molecular weight (approximately 43,ØØØ vs 42,ØØØ) reflecting the addition of 11 amino acid residues to the amino-terminus of the fusion protein made in E. coli. Apparently, these additional amino acid residues do not inactivate the inhibitor.

In addition, all of the material in the homogenates giving a positive result by the ELISA and elastase tests may be removed by the immunoadsorption procedure.

## Claims

1. A method for producing a polypeptide having the protease inhibition activity of mammalian alpha-1-antitrypsin comprising the step of growing a bacterial culture transformed by a DNA transfer vector derived from an M13 cloning vector, said transfer vector controlled by a trp-lac promoter or lac promoter being and comprising a segment coding for mammalian alpha-1-antitrypsin.

2. A method of forming a bacterium having the capability of producing a polypeptide having the protease inhibition activity of mammalian alpha-1-antitrypsin, which method comprises introducing a DNA transfer vector derived from an M13 cloning vector, comprising a trp-lac promoter and a segment coding for mammalian alpha-1-antitrypsin into a host bacterium to transform said bacterium thereby altering its genotype to encode in expressible format for said polypeptide, whereby expression of said polypeptide in said bacterium is controlled by said trp-lac promoter.

3. A method as claimed in claim 2, wherein said transfer vector has been produced by altering a suitable vector to include said segment coding for mammalian alpha-1-antitrypsin.

4. A method as claimed in any one of claims 1 to 3, wherein said culture/bacterium is E.coli.

5. A method as claimed in any one of claims 1 to 4 wherein said trp-lac promoter is derived from plasmid pDR540.

6. A method as claimed in claim 1, wherein said transfer vector is vector M13$\alpha_1$AT.

7. A method of constructing a DNA vector capable in bacteria of expressing alpha-1-antitrypsin, comprising the step of inserting a DNA segment comprising a structural gene encoding alpha-1-antitrypsin into a transfer vector derived from an M13 cloning vector, said transfer vector comprising a bacterial origin of replication, together with appropriately positioned translational start and stop signals and a trp-lac promoter segment which controls expression by said vector in bacteria of said alpha-1-antitrypsin.

8. A method as claimed in claim 8, wherein said trp-lac promoter is derived from plasmid pDR540.

9. Vector M13a$_1$AT(ATTC 40132).

5

10. A bacterium which has been transformed by a DNA transfer vector derived from an M13 cloning vector, comprising a segment coding for mammalian alpha-1-antitrypsin and a trp-lac promoter segment, whereby the genotype of said bacterium encodes in expressible format for alpha-1-antitrypsin.

11. E.coli transformed by vector M13a₁AT.

12. A method of producing a therapeutic formulation for treating disease states related to inadequate amounts of alpha-1-antitrypsin in a mammal comprising:
   growing a bacterial culture transformed by a DNA transfer vector derived from an M13 cloning vector, controlled by a trp-lac promoter or lac promoter, said vector comprising a segment coding for mammalian alpha-1-antitrypsin; and formulating the polypeptide produced by the bacterial culture for such treatment.

13. A method as claimed in claim 12, wherein the polypeptide is formulated for administration by inhalation or for intravenous administration.

## Revendications

1. Procédé de production d'un polypeptide ayant l'activité d'inhibition des protéases de l'alpha-1-antitrypsine de mammifères, comprenant les étapes consistant à cultiver une culture bactérienne transformée par un vecteur de transfert d'ADN dérivé d'un vecteur de clonage M13, ledit vecteur de transfert étant sous le contrôle d'un promoteur trp-lac ou d'un promoteur lac et comprenant un segment codant pour l'alpha-1-antitrypsine de mammifères.

2. Procédé de formation d'une bactérie présentant la capacité de production d'un polypeptide ayant l'activité d'inhibition des protéases de l'alpha-1-antitrypsine de mammifères, procédé qui consiste à introduire un vecteur de transfert d'ADN dérivé d'un vecteur de clonage M13, comprenant un promoteur trp-lac et un segment codant pour l'alpha-1-antitrypsine de mammifères, dans un hôte consistant en une bactérie pour la transformation de ladite bactérie, ce qui permet la modification de son génotype pour le codage dans un format apte à l'expression dudit polypeptide, l'expression dudit polypeptide dans ladite bactérie étant ainsi sous le contrôle dudit promoteur trp-lac.

3. Procédé suivant la revendication 2, dans lequel le vecteur de transfert a été produit par modification d'un vecteur convenable, de sorte que ce vecteur renferme le segment codant pour l'alpha-1-antitryspine de mammifères.

4. Procédé suivant l'une quelconque des revendications 1 à 3, dans lequel la bactérie en culture est E. coli.

5. Procédé suivant l'une quelconque des revendications 1 à 4, dans lequel le promoteur trp-lac est dérivé du plasmide pDR540.

6. Procédé suivant la revendication 1, dans lequel le vecteur de transfert est le vecteur M13α₁AT.

7. Procédé de construction d'un vecteur d'ADN capable d'effectuer dans des bactéries l'expression de l'alpha-1-antitrypsine, comprenant les étapes consistant à insérer un segment d'ADN comprenant un gène structural codant pour l'alpha-1-antitrypsine dans un vecteur de transfert dérivé d'un vecteur de clonage M13, ledit vecteur de transfert comprenant une origine bactérienne de réplication, conjointement avec des signaux de départ et d'arrêt de traduction positionnés de la manière appropriée et un promoteur trp-lac qui contrôle l'expression par ledit vecteur dans des bactéries de ladite alpha-1-antitrypsine.

8. Procédé suivant la revendication 8, dans lequel le promoteur trp-lac est dérivé du plasmide pDR540.

9. Vecteur M13α₁AT (ATTC 40132).

10. Bactérie qui a été transformée par un vecteur de transfert d'ADN dérivé d'un vecteur de clonage M13, comprenant un segment codant pour l'alpha-1-antitrypsine de mammifères et un promoteur trp-lac, le

EP 0 137 633 B1

génotype de ladite bactérie codant ainsi dans un format apte à l'expression de l'alpha-1-antitrypsine.

11. E. coli transformée par le vecteur M13α₁AT.

12. Procédé de production d'une formulation thérapeutique pour le traitement d'états pathologiques en rapport avec des quantités inadéquates d'alpha-1-antitrypsine chez un mammifère, consistant :
à faire croître une culture bactérienne transformée par un vecteur de transfert d'ADN dérivé d'un vecteur de clonage M13, sous le contrôle d'un promoteur trp-lac ou d'un promoteur lac, ledit vecteur comprenant un segment codant pour l'alpha-1-antitrypsine de mammifères ; et à formuler le polypeptide produit par la culture bactérienne pour un tel traitement.

13. Procédé suivant la revendication 12, dans lequel le polypeptide est formulé pour l'administration par inhalation ou pour l'administration par voie intraveineuse.

## Patentansprüche

1. Verfahren zur Herstellung eines Polypeptids mit der Protease-Hemmungsaktivität von alpha-1-Antitrypsin von Säugetieren, das den Schritt des Züchtens einer Bakterienkultur umfaßt, die mit einem von einem M13-Klonierungsvektor abgeleiteten DNA-Transfervektor transformiert ist, wobei besagter Transfervektor von einem trp-lac-Promotor oder einem lac-Promotor kontrolliert wird und ein Segment umfaßt, das für alpha-1-Antitrypsin von Säugetieren kodiert.

2. Verfahren zur Bildung eines Bakteriums mit der Fähigkeit, ein Polypeptid mit der Protease-Hemmungsaktivität von alpha-1-Antitrypsin von Säugetieren zu produzieren, wobei das Verfahren das Einführen eines von einem M13-Klonierungsvektor abgeleiteten DNA-Transfervektors, der einen trp-lac-Promotor und ein Segment umfaßt, das für alpha-1-Antitrypsin von Säugetieren kodiert, in ein Wirtsbakterium umfaßt, um besagtes Bakterium zu transformieren, wodurch sein Genotyp verändert wird, um in exprimierbarem Format für besagtes Polypeptid zu kodieren, wobei die Expression besagten Polypeptids in besagtem Bakterium von besagtem trp-lac-Promotor kontrolliert wird.

3. Verfahren nach Anspruch 2, dadurch gekennzeichnet, daß besagter Transfervektor durch Verändern eines geeigneten Vektors hergestellt worden ist, um besagtes Segment, das für alpha-1-Antitrypsin von Säugetieren kodiert, einzuschließen.

4. Verfahren nach einem der Ansprüche 1 bis 3, dadurch gekennzeichnet, daß besagte Kultur/besagtes Bakterium E. coli ist.

5. Verfahren nach einem der Ansprüche 1 bis 4, dadurch gekennzeichnet, daß besagter trp-lac-Promotor von Plasmid pDR540 abgeleitet ist.

6. Verfahren nach Anspruch 1, dadurch gekennzeichnet, daß besagter Transfervektor Vektor M13α₁AT ist.

7. Verfahren zur Konstruktion eines DNA-Vektors, der in der Lage ist, alpha-1-Antitrypsin in Bakterien zu exprimieren, das den Schritt des Inserierens eines DNA-Segmentes, das ein alpha-1-Antitrypsin kodierendes Strukturgen umfaßt, in einen von einem M13 Klonierungsvektor abgeleiteten Transfervektor umfaßt, wobei besagter Transfervektor einen bakteriellen Replikationsursprung umfaßt, zusammen mit geeignet angeordneten Translations-Start- und -Stop-Signalen und einem trp-lac-Promotor-Segment, das die Expression von besagtem alpha-1-Antitrypsin durch besagten Vektor in Bakterien kontrolliert.

8. Verfahren nach Anspruch 7, dadurch gekennzeichnet, daß besagter trp-lac-Promotor von Plasmid pDR540 abgeleitet ist.

9. Vektor M13α₁AT (ATTC 40132).

10. Bakterium, das mit einem von einem M13-Klonierungsvektor abgeleiteten DNA-Transfervektor transformiert worden ist, der ein Segment, das alpha-1-Antitrypsin von Säugetieren kodiert, und ein trp-lac-Promotor-Segment umfaßt, wodurch der Genotyp besagten Bakteriums in exprimierbarem Format für alpha-1-Antitrypsin kodiert.

7

11. E. coli, transformiert durch Vektor M13$\alpha_1$AT.

12. Verfahren zur Herstellung einer therapeutischen Zusammensetzung zur Behandlung von Erkrankungszuständen, die mit ungenügenden Mengen an alpha-1-Antitrypsin in Zusammenhang stehen, in einem Säugetier, das folgendes umfaßt:

   Züchten einer Bakterienkultur, die mit einem von einem M13-Klonierungsvektor abgeleiteten DNA-Transfervektor transformiert ist, kontrolliert von einem trp-lac-Promotor oder lac-Promotor, wobei besagter Vektor ein Segment umfaßt, das für alpha-1-Antitrypsin von Säugetieren kodiert; und Formulieren des von der Bakterienkultur produzierten Polypeptids für eine solche Behandlung.

13. Verfahren nach Anspruch 12, dadurch gekennzeichnet, daß das Polypeptid zur Verabreichung durch Inhalation oder zur intravenösen Verabreichung formuliert wird.

FIG—1

FIG.—2

EP 0 137 633 B1

Bam                    Bam

Met

CACAACAGGAAACAGGATCCATGGAGGATCC

NcoI

FUSION OF TAC
AND M13 FRAGMENTS
TAC FRAGMENT AND
mpIOB FRAGMENT

LIGATE →

mpIOC

XhoI   TAC

GGATC
CCTAG

GAT CCA TGG
CTAGGTACC

NcoI

Xho
DIGEST

GEL
PURIFY →

XhoI

GAT CCA TGG
CTAGGTACC

BamHI
PARTIAL
DIGEST

FILL IN
WITH
Klenow

LIGATE →

Xho   TAC   AGGAAACA
              TCCTTTGT

GEL
PURIFY

BamHI    FILL IN
COMPLETE    WITH
DIGEST    Klenow

ACAGGATC
TGTCCTAG

TAC

CTAGG

Xho

Bal 31
DIGEST →

ACA
TGT

TAC

XhoI
DIGEST

Xho

FIG.—3

XhoI   TAC

NcoI

BamHI

M13 TAC
( BLUE
PLAQUE )

# FIG. 4

5' GGGGGGGGGGGGGGG CA CCA CCA CTG ACC
                   10         20

```
                    -24                 -20                                          -10
                    Met Pro Ser Ser Val Ser Trp Gly Ile Leu Leu Leu Ala Gly Leu
TGG GAC AGT GAA TCG ACA ATG CCG TCT TCT GTC TCG TGG GGC ATC CTC CTG CTG GCA GGC CTG
 30          40          50          60          70          80          90

                            -1  +1                                         10
Cys Cys Leu Val Pro Val Ser Leu Ala Glu Asp Pro Gln Gly Asp Ala Ala Gln Lys Thr Asp
TGC TGC CTG GTC CCT GTC TCC CTG GCT GAG GAT CCC CAG GGA GAT GCT GCC CAG AAG ACA GAT
         100         110         120         130         140         150

                        20                                          30
Thr Ser His His Asp Gln Asp His Pro Thr Phe Asn Lys Ile Thr Pro Asn Leu Ala Glu Phe
ACA TCC CAC CAT GAT CAG GAT CAC CCA ACC TTC AAC AAG ATC ACC CCC AAC CTG GCT GAG TTC
    160         170         180         190         200         210

                    40                                          50
Ala Phe Ser Leu Tyr Arg Gln Leu Ala His Gln Ser Asn Ser Thr Asn Ile Phe Phe Ser Pro
GCC TTC AGC CTA TAC CGC CAG CTG GCA CAC CAG TCC AAC AGC ACC AAT ATC TTC TTC TCC CCA
220         230         240         250         260         270         280

                    60                                          70
Val Ser Ile Ala Thr Ala Phe Ala Met Leu Ser Leu Gly Thr Lys Ala Asp Thr His Asp Glu
GTG AGC ATC GCT ACA GCC TTT GCA ATG CTC TCC CTG GGG ACC AAG GCT GAC ACT CAC GAT GAA
        290         300         310         320         330         340

                80                                          90
Ile Leu Glu Gly Leu Asn Phe Asn Leu Thr Glu Ile Pro Glu Ala Gln Ile His Glu Gly Phe
ATC CTG GAG GGC CTG AAT TTC AAC CTC ACG GAG ATT CCG GAG GCT CAG ATC CAT GAA GGC TTC
        350         360         370         380         390         400

                100                                         110
Gln Glu Leu Leu Arg Thr Leu Asn Gln Pro Asp Ser Gln Leu Gln Leu Thr Thr Gly Asn Gly
CAG GAA CTC CTC CGT ACC CTC AAC CAG CCA GAC AGC CAG CTC CAG CTG ACC ACC GGC AAT GGC
    410         420         430         440         450         460             470

                120                                         130
Leu Phe Leu Ser Glu Gly Leu Lys Leu Val Asp Lys Phe Leu Glu Asp Val Lys Lys Leu Tyr
CTG TTC CTC AGC GAG GGC CTG AAG CTA GTG GAT AAG TTT TTG GAG GAT GTT AAA AAG TTG TAC
            480         490         500         510         520         530

            140                                         150
His Ser Glu Ala Phe Thr Val Asn Phe Gly Asp Thr Glu Glu Ala Lys Lys Gln Ile Asn Asp
CAC TCA GAA GCC TTC ACT GTC AAC TTC GGG GAC ACC GAA GAG GCC AAG AAA CAG ATC AAC GAT
            540         550         560         570         580         590

160                                         170                                 180
Tyr Val Glu Lys Gly Thr Gln Gly Lys Ile Val Asp Leu Val Lys Glu Leu Asp Arg Asp Thr
TAC GTG GAG AAG GGT ACT CAA GGG AAA ATT GTG GAT TTG GTC AAG GAG CTT GAC AGA GAC ACA
    600         610         620         630         640         650

                                    190                                 200
Asp Thr Glu Glu Glu Asp Phe His Val Asp Gln Val Thr Thr Val Lys Val Pro Met Met Lys
GTT TTT GCT CTC GTG AAT TAC ATC TTC TTT AAA GGC AAA TGG GAG AGA CCC TTT GAA GTC AAG
660         670         680         690         700         710         720

                            210                                 220
Asp Thr Glu Glu Glu Asp Phe His Val Asp Gln Val Thr Thr Val Lys Val Pro Met Met Lys
GAC ACC GAG GAA GG GAC TTC CAC GTG GAC CAG GTG ACC ACC GTG AAG GTG CCT ATC ATG AAG
            730         740         750         760         770         780

                    230                                 240
Arg Leu Gly Met Ile Asn Ile Gln His Cys Lys Lys Leu Ser Ser Trp Val Leu Leu Met Lys
CGT TTA GGC ATG ATT AAC ATC CAG CAC TGT AAG AAG CTG TCC AGC TGG GTC CTG CTG ATG AAA
        790         800         810         820         830         840
```

# FIG. 4 page 2

```
                          250                                    260
Tyr Leu Gly Asn Ala Thr Ala Ile Phe Phe Leu Pro Asp Glu Gly Lys Leu Gln His Leu Glu
TAC CTG GGC AAT GCC ACC GCC ATC TTC TTC CTG CCT GAT GAG GGG AAA CTA CAG CAC CTG GAA
850          860         870         880         890         900         910


                       270                                  280
Asn Glu Leu Thr His Asp Ile Ile Thr Lys Phe Leu Glu Asn Glu Asp Arg Arg Ser Ala Ser
AAT GAA CTC ACC CAC GAT ATC ATC ACC AAG TTC CTG GAA AAT GAA GAC AGA AGG TCT GCC AGC
             920         930         940         950         960         970


                    290                                  300
Leu His Leu Pro Lys Leu Ser Ile Thr Gly Thr Tyr Asp Leu Lys Ser Val Leu Gly Gln Leu
TTA CAT TTA CCC AAA CTG TCC ATT ACT GGA ACC TAT GAT CTG AAG AGC GTC CTG GGT CAA CTG
          980         990         1000        1010        1020        1030


                 310                                  320
Gly Ile Thr Lys Val Phe Ser Asn Gly Ala Asp Leu Ser Gly Val Thr Glu Glu Ala Pro Leu
GGC ATC ACT AAG GTC TTC AGC AAT GGG GCT GAC CTC TCC GGG GTC ACA GAG GAG GCA CCC CTG
  1040        1050        1060        1070        1080        1090        1100


              330                                  340                        350
Lys Leu Ser Lys Ala Val His Lys Ala Val Leu Thr Ile Asp Glu Lys Gly Thr Glu Ala Ala
AAG CTC TCC AAG GCC GTG CAT AAG GCT GTG CTG ACC ATC GAC GAG AAA GGG ACT GAA GCT GCT
          1110        1120        1130        1140        1150        1160


                                               360                        370
Gly Ala Met Phe Leu Glu Ala Ile Pro Met Ser Ile Pro Pro Glu Val Lys Phe Asn Lys Pro
GGG GCC ATG TTT TTA GAG GCC ATA CCC ATG TCT ATC CCC CCC GAG GTC AAG TTC AAC AAA CCC
          1170        1180        1190        1200        1210        1220


                                            380                             390
Phe Val Phe Leu Met Ile Glu Gln Asn Thr Lys Ser Pro Leu Phe Met Gly Lys Val Val Asn
TTT GTC TTC TTA ATG ATT GAA CAA AAT ACC AAG TCT CCC CTC TTC ATG GGA AAA GTG GTG AAT
  1230        1240        1250        1260        1270        1280


              394
  Pro Thr Gln Lys STOP
  CCC ACC CAA AAA TAA CTG CCT CTC GCT CCT CAA CCC CTC CCC TCC ATC CCT GGC CCC CTC CCT
1290        1300        1310        1320        1330        1340        1350



GGA TGA CAT TAA AGA AGG GTT GAG CTG
     1360        1370



  G AAAAAAAAAAAAAAAA CCCCCCCCCCCCCCCCCCCCCCCCCCCCCCCCCCCCCCCCCC 3'
1380      1390      1400      1410      1420      1430
```

```
                                      1   2   3   4   5   6   7   8   9   10  11  12  13  14  15  16
                  1   2   3   4   PRO SER LEU GLY CYS ARG SER THR LEU GLU ASP PRO ARG ALA SER SER  5   6   7   8
M13mpII/pUCI3    THR MET ILE THR                                                                 ASN SER LEU ALA
      ATG ACC ATG ATT ACG CCA AGC TTG GGC TGC AGG TCG ACT CTA GAG GAT CCC CGG GCG AGC TCG AAT TCA CTG GCG
                  HindIII         PstI      SalI      XbaI      BamHI  XmaI       SatI     EcoRI          HaeIII
                                            AccI,HincII                Smal
```

# FIG.—5A

EP 0 137 633 B1

FIG.—5B

FIG. 6

FIG.—7